# EUROPEAN PATENT SPECIFICATION

(11) **EP 3 656 303 B1**
(45) Date of publication and mention of the grant of the patent: **30.06.2021**
(21) Application number: 18207772.7
(22) Date of filing: 22.11.2018
(51) Int. Cl.: A61B 5/15, A61B 5/151

(54) **BLOOD WITHDRAWAL SYSTEM WITH LANCET EJECTION MECHANISM**
BLUTENTNAHMESYSTEM MIT LANZETTENAUSSTOSSMECHANISMUS
SYSTÈME DE RETRAIT DE SANG DOTÉ D'UN MÉCANISME D'ÉJECTION DE LANCETTE

(43) Date of publication of application: 27.05.2020
(73) Proprietor: Roche Diabetes Care GmbH, 68305 Mannheim (DE); F. Hoffmann-La Roche AG, 4070 Basel (CH)
(72) Inventor: Wiegand, Roland, 68305 Mannheim (DE)
(74) Representative: Rentsch Partner AG

(56) References cited:
- EP-A1- 0 428 914
- WO-A2-2008/052172
- US-A- 4 442 836
- US-A1- 2010 049 092
- US-A1- 2013 274 780
- US-A1- 2013 338 537

## Description

### Field of disclosure

The present invention lies in the field of blood withdrawal and blood analysis. More particularly, it is related to a blood withdrawal system with a base device configured to hold a structurally distinct lancet. The base device comprises a lancet ejection mechanism. Furthermore, the invention is directed to a method for ejecting a lancet from a basic device of a blood withdrawal system.

### Background, prior art

Typically, blood withdrawal systems comprise small lancets, which are used to generate a wound in the patient's tissue for withdrawing a blood sample of small quantity. Devices known in the art in large variety comprise a housing with a lancet drive including a drive element, which is typically a spring. At first, these blood withdrawal systems are brought in an initial state, in which the spring is tensed. The system is then positioned on the patient's tissue and the spring is released, thus forcing the lancet into the tissue. For hygienic reasons, the lancet is only used a single time and disposed thereafter.

Blood withdrawal systems for withdrawing small blood samples may be particularly used by patients suffering from diabetes mellitus. In order to reduce the risk of severe consequences, diligent diabetes therapies require at least four analyses per day. In an attempt to reduce the number of lancet exchanging events, blood withdrawal systems have been developed which include lancet magazines. These magazines are capable of housing several lancets, which may be automatically or semi-automatically exchanged. For example, a magazine can comprise five lancets which are arranged in a circular manner. After a lancet has been used, an exchange mechanism is triggered, upon which the used lancet is returned to the magazine, and the magazine is rotated such that a novel lancet can be used for the next blood withdrawal event. After all lancets in the magazine have been used, an ejection mechanism is triggered by the user, in which the magazine is ejected and subsequently exchanged for a new one.

In order to reduce the risk of unintentional injuries, the lancets of blood withdrawal systems are usually located within its housing and only protrude upon activation of the lancet drive for puncturing the finger and are subsequently withdrawn back into the housing. Therefore, blood withdrawal systems in general comprise a housing with an exit opening for at least the tip of the lancet. During puncturing, the lancet can typically be moved relative to the housing, along a predetermined straight puncturing path by the lancet drive. The lancet drive is usually based on an elastic drive element such as a metal spring. As mentioned above, the spring is tensed in an initial state and usually kept in this state by a locking element. The locking element can then be released by the user, for example by pushing a button, upon which a puncturing movement of the lancet drive occurs, which moves the lancet with high speed along the predetermined puncturing path until at least the tip of the lancet protrudes from the housing through the exit opening. In other embodiments, tensioning the spring and subsequently triggering the lancet movement are done via a single user operation. If the blood withdrawal system is correctly positioned on the patient's tissue, a wound is thereby generated and the lancet is subsequently withdrawn. In the following, the lancet or the lancet magazine is then removed from the blood withdrawal system by a lancet ejection mechanism and replaced by a new lancet or by a new lancet magazine. Alternatively, the blood withdrawal systems may be produced as single use devices. Such systems are fully disposable and thus no lancet ejection mechanism is typically necessary.

Document US 2010/049092 A1 discloses a coupling facility 7. The coupling facility 7 grasps the lancet carrier 4 in a clamping manner during a puncture and releases it again after a puncturing and returning motion are completed. Accordingly, FIG. 7 shows the coupling facility 7 before a puncture with a released lancet carrier 4. In FIG. 8, the lancet carrier 4 is clamped by the coupling facility 7, i.e. it is shown according to the conditions that are evident during a puncture. The coupling facility 7 comprises a sled 10 that can be driven in the direction of puncturing and carries a receptacle 8 through which the lancet carrier 4 is fed. The clamping force for clamping the lancet carrier 4 is effected by a spring element 14. The spring element 14 interacts with an opener 17 that is arranged fixed in place with respect to the magazine housing in the form of a projection on the magazine housing. If the sled 10 is in the position shown in FIG. 7 after a puncture, the opener 17 presses against the spring element 14 and by this means causes the receptacle 8 to open so the lancet can be ejected.

### Summary of disclosure

In general, single use blood withdrawal systems are not desirable. Not only are these systems associated with high overall cost for the user in the long term, but they also produce a significant amount of waste, in particular plastic waste.

Consequently, blood withdrawal systems in which only the lancet is disposed and replaced by a new one are preferred. In particular, such devices may already include an analytical device, such as a sensor, for testing the blood sample either directly or via insertion of a sensor strip.

As described above, these systems typically require an additional lancet ejection mechanism. In this respect, it is irrelevant whether a single lancet or a lancet magazine is employed. The additional lancet ejection mechanism however has a deleterious effect on the overall cost of the system. Such additional ejection mechanisms are typically realized by implementing a specific slider or button as part of a user interface. In addition, the button has to be coupled to the lancet, which makes the setup of the blood withdrawal system significantly more complicated.

Furthermore, any additional part represents a potential source for malfunction of the blood withdrawal system. This is especially the case for mass product devices, which have to be manufactured in a simple and cheap manner. Besides the fact that this can lead to less robust blood withdrawal systems, such malfunctions can have severe consequences for the user. If for example the additional lancet ejection mechanism fails, the user may attempt to remove the lancet manually and potentially injure himself in the event.

It is therefore an overall objective of the present invention to improve the state of the art regarding the ejection of lancets in blood withdrawal systems, thereby preferably avoiding disadvantages of the prior art fully or partly. In particular, the blood withdrawal systems may be used for the monitoring of the blood glucose level.

In favorable embodiments, the blood withdrawal system can be manufactured in a cost efficient way.

In further favorable embodiments, a blood withdrawal system with a lancet ejection mechanism is provided, whose setup is essentially not more complicated than the setup of a single use blood withdrawal system without a lancet ejection mechanism.

In another favorable embodiment, a secure and reliable blood withdrawal system and method for ejecting a lancet from a basic device of a blood withdrawal system is provided with a straightforward lancet ejection mechanism, which preferably reduces unintentional injuries of the patient.

The overall object is in a general way achieved by the subject of the independent claims. Further advantageous embodiments follow from the dependent claims, the description and the figures.

According to a first aspect, the overall objective is achieved by a blood withdrawal system for diagnostic purposes, comprising a base device configured to hold a structurally distinct lancet. The base device includes an actuatable lancet ejection mechanism for ejecting the lancet from the base device, wherein the lancet ejection mechanism comprises a movable coupling element. Furthermore, the movable coupling element comprises a coupling structure for coupling the lancet with an external component via the coupling element and subsequently activating the lancet ejection mechanism by operating the external component.

It is to be understood herein that an external component refers to a component which is not part of and is distinct from the blood withdrawal system. The external component may be operated by the user, that is, the external component may be user activated and/or user controlled. Additionally, the movable coupling element may be accessible by the external component. The fact that the movable coupling element comprises a coupling structure for coupling the lancet with the external component via the coupling element provides the advantage that no additional slider, button or additional input means have to be provided. As the coupling element is configured to activate the lancet ejection mechanism, the ejection of the lancet can be achieved with a blood withdrawal system that requires only a minimum number of components and thus both the use and the manufacture of the system are significantly simplified.

A lancet typically comprises a sharp blade or needle and is used to generate the puncture in the patient's tissue. Furthermore, the lancet may comprise a lancet holder, which may for example be a plastic component at least partially surrounding the lancet for securing the lancet within the basic device of the blood withdrawal system.

As the skilled person understands, the ejection of the lancet may for example be achieved by pushing the lancet out of the basic device or simply by turning the system such that the lancet drops out of the basic device solely due to the gravitational force.

The movable coupling element may be a coupling element, which may for example be rotated, sheared, and/or linearly moved within the blood withdrawal system. Typically, the coupling element is made from a single part. For example, the coupling element may be an injection-molded single element. As an example, the coupling element may be a rocker.

In typical embodiments, the base device comprises a lancet receptacle for holding the lancet. Such a lancet receptacle may be releasably connected to the lancet by way of friction and/or force locking. For example, the lancet may at least be connected to the lancet receptacle until the piercing event has taken place. Thus, in typical embodiments, the lancet receptacle is movable in the piercing direction, such that the lancet receptacle guides the lancet during the puncturing event. After the piercing event, the connection between the lancet receptacle and the lancet may be released.

Preferably, the blood withdrawal system further comprises a drive mechanism, which is typically spring operated. The drive mechanism is configured to move the lancet along a predetermined path into a protruding position such that a wound can be generated in the patient's skin. Furthermore, the drive mechanism may also be configured to withdraw the lancet after wound generation.

In further embodiment, the blood withdrawal system further comprises the structurally distinct lancet.In an embodiment, the basic device of the blood withdrawal system further comprises a housing, wherein the housing encloses the ejection mechanism. The housing further comprises a receiving aperture being configured to receive a portion of the external component. The receiving aperture may for example be an opening for the external component, such as a slot, cavity, groove, recess, hole, gap, etc. Typically, the aperture may extend towards the coupling element. Thus, the coupling element is configured to be coupled with the external component. In particular, the movable coupling element may be configured to at least partially transfer a force of the external component acting on the coupling element to the lancet. In particular after receiving a portion of the external component, the remaining portion of the external component may be operated by the user for activating the lancet ejection mechanism. For example, the user may exert a linear force on the external component, i.e. push the external component towards the coupling element or the user may rotate the external component.

Preferably, the receiving aperture directly extends to the coupling element. That is, the coupling element is directly accessible by the external component.

In some embodiments, the housing may further comprise a lancet opening through which the lancet can protrude for penetrating the skin or through which the lancet can be ejected for disposal.

In a further embodiment, the coupling element comprises a pivot to redirect an input linear or rotational movement and/or to transform an input linear movement of the external component into a rotational or sheared movement of the coupling element. As a result, the coupling element may be designed in a simplified manner and thus the whole blood withdrawal system may possess a simple and easy to manufacture design. Furthermore, due to the simple design, the probability for any malfunctions are significantly decreased.

In typical embodiments, the pivot may be configured to enable a rotational or sheared movement of at least parts of the coupling element. In particular, the pivot may be a hinge joint, film hinge, swivel joint, a flexible structure or any other suitable joint.

Furthermore, the coupling element of the blood withdrawal system may comprise two arms, which are connected with each other via the pivot in an angle α. The angle α may for example be 180°. In this case, the coupling element may be a straight bar, for example a rocker. Such a bar may preferably be rotatable around the pivot. The coupling element may also be V-shaped, i.e. the angle α may be between 0 and 180°, such as 120° or 135°. The angle α may also be 90°. In this case, the coupling element may be L-shaped. Furthermore, the coupling element may comprise more than two arms. For example, a first arm may be connected to a second arm in a first angle α₁ and the second arm may be connected to a third arm in a second angle α₂.

In preferred embodiments in which the coupling element comprises two or more arms, one arm may operatively couple respectively interact with the lancet in its initial configuration prior to ejection. For example the arm may be pressed against the lancet such that a force is exerted on the lancet and/or engage with an opening or aperture of the lancet.

In another embodiment, the blood withdrawal system further comprises a biasing element. The biasing element exerts an initial force on the lancet in its initial configuration, wherein the initial force is reduced or removed upon activation of the lancet ejection mechanism. The biasing element may be a spring made of metal or an elastic polymer, such as a rubber or silicone block. In certain preferred embodiments, the biasing element may operatively couple and interact, with one arm of the coupling element in a way that the arm of the coupling element is pushed towards the lancet. As a result, a force may be exerted on the lancet such that the lancet is clamped between the coupling element and the housing or any other counterpart of the basic device. Therefore, the initial force of the biasing element exerted on the lancet does not necessarily need to be exerted directly on the lancet. In other embodiments, the biasing element may directly be in contact with the lancet and thus directly exert an initial force on the lancet. In such embodiments, activation of the lancet ejection mechanism includes the release of the interaction between the coupling element and the lancet upon which the initial force of the biasing element exerted on the lancet is reduced as the lancet is pushed out of the basic device.

In a preferred embodiment, the coupling element comprises a coupling structure for coupling the lancet with an external component, wherein the external component is, for example, a test strip or an ejection pin. If the external component is a test strip, the ejection of the lancet by coupling the test strip with the lancet via the coupling element and subsequent activation of the lancet ejection mechanism by operating the external component may be performed concomitantly with an analysis of the test strip by an analytical device in the blood withdrawal system. As a result, such a blood withdrawal system is essentially not more complex than a single use blood withdrawal system. In particular, the receiving aperture may concomitantly be used for coupling the test strip with the lancet and subsequently activate the lancet ejection mechanism as well as for analyzing the blood on the test strip. In some embodiments, the coupling structure may be designed to couple with an external component, in particular a pin-shaped or elongated component, of different type that may serve as ejection pin, such as a toothpick, a match or a piece of wire, e. g. an end section of a paper clip.

It is clear to the skilled person that the test strip or ejection pin must be sufficiently stiff for activating the lancet ejection mechanism. Typically test strips which are known in the art and which are suitable for the purpose described herein may for example be cuboid plastic strips with a thickness of 0.5 to 2 mm.

According to a further aspect of the invention, the overall technical problem is solved by a kit of parts comprising:
- the embodiment of a blood withdrawal system described above wherein the coupling element comprises a coupling structure for coupling the lancet with an external component, wherein the external component is a test strip or an ejection pin; and
- a test strip or an ejection pin.

According to a further aspect of the invention, the overall technical problem is solved by a method for ejecting a lancet from a basic device of a blood withdrawal system comprising the steps of coupling an external component with the lancet via a movable coupling element; activating a lancet ejection mechanism by operating the external component; and ejecting the lancet.

As already described above, the external component may typically be a test strip or an ejection pin, such as a toothpick, a match or any other element suitable for that purpose.

In a preferred embodiment the coupling element transforms an input force into an output force, which acts on the lancet or on a biasing element.

Typically the biasing element may be a spring or a flexible polymer, as described above.

In a further embodiment, a first arm of the coupling element is moved in a first direction and a second arm of the coupling element is moved in a second direction. For example, the first and the second direction may be the same, i.e. the pathways of movement may be parallel or both be clockwise rotations. Alternatively, the first and second direction may be different from each other, i.e. the pathways of movement are different.

It is understood that moving in a direction includes both linear movements, for example a linear movement towards the coupling element, as well as rotational movements, for example in a clockwise or counterclockwise direction.

In another embodiment the coupling element reduces or removes an initial force exerted by a biasing element on the lancet in its initial configuration upon activation of the lancet ejection mechanism.

For example, a method according to such an embodiment may comprise the steps:
i) coupling an external component with the lancet via a movable coupling element;
ii) activating a lancet ejection mechanism by operating the external component;
iii) reducing or removing an initial exerted by a biasing element on the lancet in its initial configuration;
iv) ejecting the lancet.

### Brief description of the figures

Figure 1 shows a schematic, cross-sectional view of a blood withdrawal system for diagnostic purposes in accordance to one embodiment of the invention.
Figure 2 shows a schematic, cross-sectional view of a blood withdrawal system for diagnostic purposes in accordance to another embodiment of the invention.
Figure 3 shows a schematic, cross-sectional view of a blood withdrawal system for diagnostic purposes in accordance to another embodiment of the invention.
Figure 4 shows a schematic, cross-sectional view of a blood withdrawal system for diagnostic purposes in accordance to another embodiment of the invention.
Figure 5 shows a schematic, cross-sectional view of a blood withdrawal system for diagnostic purposes in accordance to another embodiment of the invention.
Figure 6 shows a schematic, cross-sectional view of a blood withdrawal system for diagnostic purposes in accordance to another embodiment of the invention.

### Exemplary embodiments

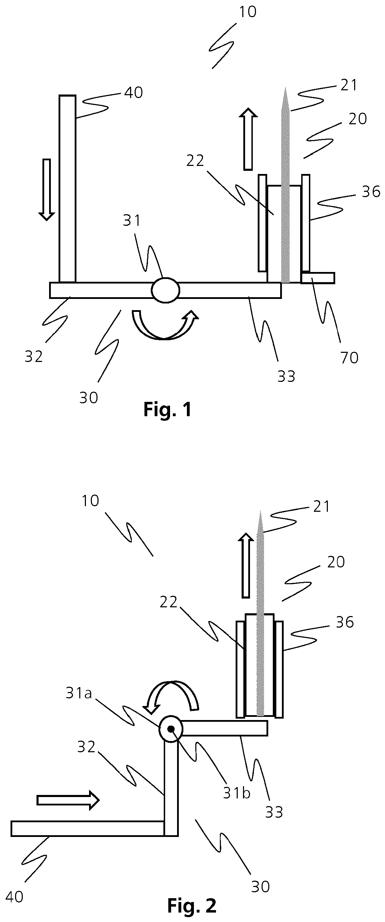

An advantageous embodiment of a blood withdrawal system 10 for diagnostic purposes is shown in **Figure 1****.** The blood withdrawal system 10 comprises a base device configured to hold a structurally distinct lancet 20, with blade 21 and lancet holder 22. The base device additionally includes lancet receptacle 36, for holding the lancet. Upon operating the blood withdrawal system, lancet receptacle 36 is operated by drive mechanism 70 (not shown in detail) and guides lancet 20 in the piercing direction into a protruding position. The drive mechanism may typically be a spring mechanism. After puncturing the skin of the patient, the drive mechanism may also be configured to withdraw the lancet and/or the lancet receptacle. Furthermore, the base device comprises an actuatable lancet ejection mechanism for removing the lancet from the base device. The lancet ejection mechanism further comprises a movable coupling element 30. Coupling element 30 comprises a coupling structure for coupling the lancet 20 with an external component 40 via the coupling element 30 and subsequently activating the lancet ejection mechanism by operating the external component. The coupling element may for example be a rocker. In the embodiment illustrated, the lancet may be ejected by operating the external component, for example by a force exerted by the coupling element on the lancet. The coupling element 30 includes a pivot 31, which may be a joint, such as a hinge joint, film hinge, swivel joint, a flexible structure or any other suitable joint. When the external component 40 is coupled with the lancet by the coupling element and operated, an input linear movement of the external component 40 can be transformed into a rotatory movement of the coupling element 30 around pivot 31, thereby exerting a force on the lancet such that the lancet is ejected from the basic device and the lancet receptacle. Furthermore, as indicated by the straight arrows, an input linear movement of the external component 40 is redirected into a linear movement of the lancet in the counter direction. The coupling element 30 further includes two arms, first arm 32 and second arm 33, which are connected with each other via pivot 31 in an angle α = 180°. As can be seen, at an angle of 180°, the coupling element may have the shape of a straight bar. In particular, such a bar may be made from a single piece. In the embodiment shown, second arm 33 contacts the lancet 20 in its initial configuration prior to ejection.

**Figure 2** shows another embodiment of a blood withdrawal system 10 for diagnostic purposes according to the invention. The blood withdrawal system comprises a base device configured to hold a structurally distinct lancet 20. The base device includes movable coupling element 30, which comprises first arm 32 and second arm 33. First arm 32 and second arm 33 are connected via pivot 31 in an angle α = 90° with each other. The pivot comprises female part 31a and male counterpart 31b. The pivot is configured to redirect an input linear movement of the external component by 90° to a linear movement of the lancet and to transform the input linear movement of the external component into a rotational movement of the coupling element. Upon coupling the external component with the lancet 20 via coupling element 30 and activating the lancet ejection mechanism, the coupling element may conduct a counterclockwise rotation around male counterpart 31b, thereby ejecting the lancet from the basic device via rotatory movement of second arm 33. Like in further subsequently described embodiments, no drive mechanism is shown for clarity reasons. However, a drive mechanism similar to the drive mechanism 70 of Figure 1 is typically present.

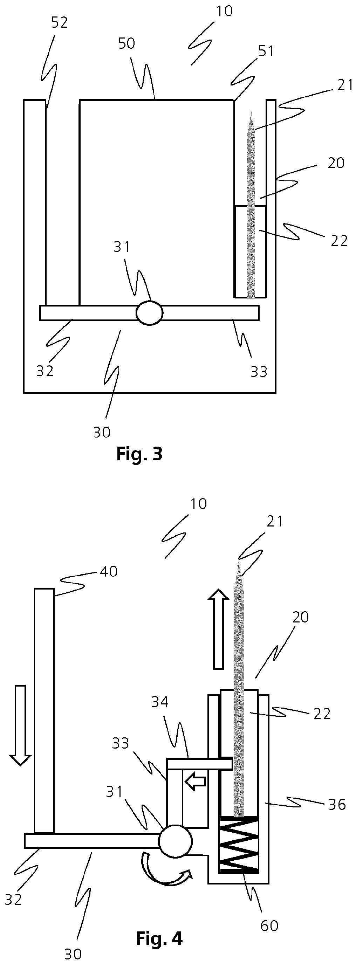

A further advantageous embodiment of a blood withdrawal system 10 according to the invention is depicted in **Figure 3****.** Besides movable coupling element 30 and structurally distinct lancet 20, Figure 3 illustrates housing 50 of the basic device enclosing the ejection mechanism. Housing 50 includes receiving aperture 52, which extends towards coupling element 30 and which is configured to receive a portion of the external component. In the embodiment shown, receiving aperture 52 is designed as an additional opening in the housing 50. Receiving aperture 52 is configured to receive the external component to enable coupling of the external component with the lancet via coupling element 30 and to subsequently activate the lancet ejection mechanism by operating the external component. Even though the coupling element 30 in the embodiment of Figure 3 is designed similar to the coupling element of Figure 1, it is clear that any other coupling element described herein is also employable together with a receiving aperture. As can be seen, lancet 20, including blade 21 is fully in its initial configuration, i.e. a retracted position in which the structurally distinct lancet is shielded by housing 50. The initial configuration may be assumed either when the blood withdrawal system has not yet been used or when the blood withdrawal system has just been used by activation of a lancet drive (not shown), which guides the lancet through lancet opening 51 in the housing and into the patient's tissue. Immediately after the wound has been generated, the lancet is retracted by the lancet drive for safety reasons. The patient may now contact a test strip with the blood exiting the wound. In particularly preferred embodiments, blood withdrawal system 10 includes an analytic device, such as a sensor, for analyzing the blood obtained from the wound. The patient may therefore insert the test strip, i.e. an external component, into receiving aperture 52, for both analyzing the blood and also coupling the test strip with lancet 20 via the coupling element and subsequently activate the lancet ejection mechanism by operating the external component which entails the ejection of the lancet from the basic device.

**Figure 4** shows another embodiment of a blood withdrawal system 10 according to the invention with structurally distinct lancet 20 including blade 21 and lancet holder 22. The system 10 further comprises a base device with an actuatable lancet ejection mechanism with coupling element 30. As can be seen, coupling element 30 comprises pivot 31, around which the coupling element is rotatable or at which the coupling element may be sheared. The coupling element additionally comprises first arm 32, second arm 33 and third arm 34. The angle α₁ between first arm 32 and second arm 31 at the pivot is 90°. The angle α₂ between second arm 33 and third arm 34 is also 90°. Optionally, the coupling element may additionally comprise a second pivot between second arm 33 and third arm 34. In the particular embodiment shown it is preferred that pivot 31 is a film hinge. Third arm 34 is engaged with lancet 20 in its initial configuration prior to ejection. As indicated in Figure 4, the engagement is achieved by a recess within lancet holder 22, which is configured to receive at least a portion of third arm 34. Blood withdrawal system 10 further comprises lancet receptacle 36 and spring 60 as a biasing element, which exerts an initial force on lancet 20 in its initial configuration. In this embodiment, the spring is in direct contact with the lancet. When the blood withdrawal system is used for generating a wound in the patient's tissue, the lancet, the lancet receptacle as well as the coupling element may be moved in the puncturing direction (indicated by the vertical, upwards arrow) via a drive mechanism. The movement may be triggered by a drive mechanism (not shown), similar to drive mechanism 70 of Figure 1. It is further shown in Figure 4 that the lancet receptacle may be connected to pivot 31. As a consequence, lancet receptacle 36 is configured to carry pivot 31. Furthermore, figure 4 depicts the initial configuration in which the spring is tensed and blocked from releasing by the engagement of third arm 34 with lancet 20. When an external component 40, such as an ejector pin or a test strip, is coupled with lancet 20 via coupling element 30 it may exert a force on the first arm 32, which leads to a rotational and/or sheared movement of coupling element 30 around or at pivot 31, thereby enabling retraction of the third arm 34 out of the recess within the lancet holder (as indicated by the arrows), such that ultimately the engagement of third arm 34 and the lancet is lifted and the energy stored in biasing element 60 can be released and the initial force exerted on the lancet is reduced. As a result, the lancet is pushed out of the basic device.

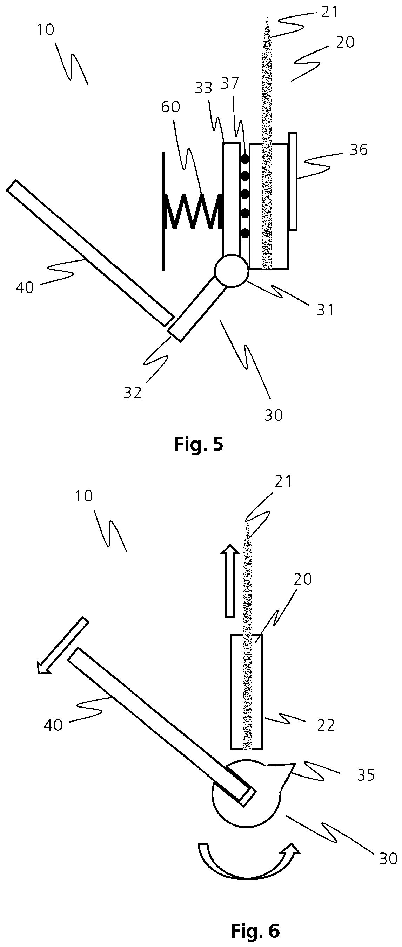

A different embodiment of a blood withdrawal system 10 with a biasing element 60 is shown in **Figure 5****.** Biasing element 60 may be an elastic or resilient element, such as a coil spring or a block made from resilient material, such as a rubber or silicone block. Biasing element 60 exerts a force on lancet 20. In contrast to the embodiment shown in Figure 4, the biasing element is not in direct contact with lancet 20. The blood withdrawal system further comprises a linear bearing 37, which may for typically be a linear ball bearing or linear friction bearing. Linear bearing 37 may be part of the coupling element 30, of lancet receptacle 36 or be an additional part of the base device. The linear bearing is configured to enable linear movement of the lancet and optionally lancet receptacle 36 in the puncturing direction to bring the lancet in a protruding position. This may for example be achieved by allowing a sliding movement of the lancet in the puncturing direction. Coupling element 30 further comprises first arm 32, which is connected with second arm 33 at pivot 31 in an angle α of approximately 165°. The movable coupling element 30 comprises a coupling structure for coupling lancet 20 with an external component 40 via the coupling element and for subsequently activating the lancet ejection mechanism. In the event, external component 40 exerts a force on first arm 32, which entails counterclockwise rotatory movement of the coupling element. As a result, the second arm compresses biasing element 60 thereby releasing lancet 20 from its initial configuration. If the user wants to remove the lancet, he may simply turn the device, such that the lancet is released solely due to gravitational force.

Another embodiment of a blood withdrawal system 10 according to the invention is shown in **Figure 6****.** System 10 comprises a base device and structurally distinct lancet 20. The base device includes an actuatable lancet ejection mechanism for ejecting the lancet from the base device with a movable coupling element 30. Coupling element 30 has an essentially circular cross-section with nose 35 and a recess or slot for receiving an external component 40. For example, coupling element 30 may be essentially disk-shaped with the exception of nose 35 and the slot or recess. As indicated by the arrows, coupling element 30 may be rotationally movable. In the particular embodiment shown, the coupling element has a pivot (not shown), to transform an input movement of the external component 40 into a linear movement of lancet 20. If the user inserts coupling element 40 into the recess, coupling element 30 is rotationally movable by turning the external component. In particular, the coupling element is configured such that nose 35 is in close proximity or already in contact with lancet 20. Therefore, operation of the external component 40 by the user (i.e. by rotating the external component) activates the lancet ejection mechanism. In particular, a force is exerted by the external component 40 on the coupling element 30 such that ultimately lancet 20 is ejected from the base device.

### List of designations

- 10: blood withdrawal system
- 20: lancet
- 21: blade
- 22: lancet holder
- 30: coupling element
- 31: pivot
- 32: first arm
- 33: second arm
- 34: third arm
- 35: nose
- 36: lancet receptacle
- 37: linear bearing
- 40: external component
- 50: housing
- 51: lancet opening
- 52: receiving aperture
- 60: biasing element
- 70: drive mechanism

## Claims

1. A blood withdrawal system (10) for withdrawing blood for diagnostic purposes, comprising:
a base device configured to hold a structurally distinct lancet (20);
the base device comprising an actuatable lancet ejection mechanism for ejecting the lancet from the base device, wherein the lancet ejection mechanism comprises a movable coupling element (30);
wherein the coupling element (30) comprises a coupling structure for coupling the lancet (20) with an external component (40) via the coupling element (30) and
subsequently activating the lancet ejection mechanism by operating the external component (40).

2. The blood withdrawal system (10) according to claim 1, wherein the blood withdrawal system further comprises the lancet (20).

3. The blood withdrawal system (10) according to any of the previous claims, wherein the base device comprises a lancet receptacle (36) for holding the lancet (20).

4. The blood withdrawal system (10) according to any of the previous claims, wherein the base device comprises a housing (50), the housing (50) enclosing the ejection mechanism, the housing (50) further comprising a receiving aperture (52), the receiving aperture (52) being configured to receive a portion of the external component.

5. The blood withdrawal system (10) according to any of the previous claims, wherein the coupling element (30) comprises a pivot (31) to redirect an input linear or rotational movement of the external component (40) and/or to transform an input linear movement of the external component (40) into a rotational or sheared movement of the coupling element (30).

6. The blood withdrawal system (10) according to claim 5, wherein the coupling element (30) comprises two arms (32, 33) wherein the arms are connected with each other via the pivot (31) in an angle α.

7. The blood withdrawal system (10) according to claim 6, wherein one arm (33, 34) operatively couples with the lancet (20) in its initial configuration prior to ejection.

8. The blood withdrawal system (10) according to any of the previous claims, further comprising a biasing element (60), wherein the biasing element (60) exerts an initial force on the lancet (20) in its initial configuration, wherein the initial force is reduced or removed upon activation of the lancet ejection mechanism.

9. The blood withdrawal system (10) according to any of the previous claims, wherein the coupling element (30) comprises a coupling structure for coupling the lancet (20) with an external component (40), the external component (40) being a test strip or an ejection pin.

10. Kit of parts comprising a blood withdrawal system according to claim 9 and a test strip or an ejection pin.

11. A method for ejecting a lancet from a basic device of a blood withdrawal system comprising the steps of:
- Coupling an external component with the lancet via a movable coupling element;
- Activating a lancet ejection mechanism by operating the external component; and
- Ejecting the lancet.

12. The method according to claim 11, wherein the coupling element transforms an input force into an output force which acts on the lancet or on a biasing element.

13. The method according to any of claims 11 or 12, wherein a first arm of the coupling element is moved in a first direction and a second arm of the coupling element is moved in a second direction.

14. The method according to any of claims 11 to 13, wherein the coupling element reduces or removes an initial force exerted by a biasing element on the lancet in its initial configuration upon activation of the lancet ejection mechanism.

## Patentansprüche

1. Blutentnahmesystem (10) zur Blutentnahme zu Diagnosezwecken, umfassend:
eine Basisvorrichtung, die so eingerichtet ist, dass sie eine strukturell separate Lanzette (20) hält;
wobei die Basisvorrichtung einen betätigbaren Lanzettenausstoßmechanismus zum Ausstoßen der Lanzette aus der Basisvorrichtung umfasst, wobei der Lanzettenausstoßmechanismus ein bewegliches Kopplungselement (30) umfasst;
wobei das Kopplungselement (30) eine Kopplungsstruktur zum Koppeln der Lanzette (20) über das Kopplungselement (30) mit einer externen Komponente (40) und anschließenden Auslösen des Lanzettenausstoßmechanismus durch Betätigen der externen Komponente (40) umfasst.

2. Blutentnahmesystem (10) nach Anspruch 1, wobei das Blutentnahmesystem ferner die Lanzette (20) umfasst.

3. Blutentnahmesystem (10) nach einem der vorhergehenden Ansprüche, wobei die Basisvorrichtung eine Lanzettenaufnahme (36) zum Halten der Lanzette (20) umfasst.

4. Blutentnahmesystem (10) nach einem der vorhergehenden Ansprüche, wobei die Basisvorrichtung ein Gehäuse (50) umfasst, wobei das Gehäuse (50) den Ausstoßmechanismus umschließt, wobei das Gehäuse (50) ferner eine Aufnahmeöffnung (52) umfasst, wobei die Aufnahmeöffnung (52) so eingerichtet ist, dass sie einen Teil der externen Komponente aufnimmt.

5. Blutentnahmesystem (10) nach einem der vorhergehenden Ansprüche, wobei das Kopplungselement (30) ein Drehgelenk (31) umfasst, um eine lineare oder rotatorische Eingangsbewegung der externen Komponente (40) umzuleiten und/oder um eine lineare Eingangsbewegung der externen Komponente (40) in eine rotatorische Bewegung oder Scherbewegung des Kopplungselements (30) umzuwandeln.

6. Blutentnahmesystem (10) nach Anspruch 5, wobei das Kopplungselement (30) zwei Arme (32, 33) umfasst, wobei die Arme über das Drehgelenk (31) unter einem Winkel α miteinander verbunden sind.

7. Blutentnahmesystem (10) nach Anspruch 6, wobei ein Arm (33, 34) vor dem Ausstoßen mit der Lanzette (20) in ihrer Ausgangskonfiguration funktionsmäßig gekoppelt ist.

8. Blutentnahmesystem (10) nach einem der vorhergehenden Ansprüche, das ferner ein Vorspannelement (60) umfasst, wobei das Vorspannelement (60) eine Anfangskraft auf die Lanzette (20) in ihrer Ausgangskonfiguration ausübt, wobei die Anfangskraft bei Auslösung des Lanzettenausstoßmechanismus verringert oder aufgehoben wird.

9. Blutentnahmesystem (10) nach einem der vorhergehenden Ansprüche, wobei das Kopplungselement (30) eine Kopplungsstruktur zum Koppeln der Lanzette (20) mit einer externen Komponente (40) umfasst, wobei die externe Komponente (40) ein Teststreifen oder ein Ausstoßstift ist.

10. Teilesatz umfassend ein Blutentnahmesystem nach Anspruch 9 und einen Teststreifen oder einen Ausstoßstift.

11. Verfahren zum Ausstoßen einer Lanzette aus einer Basisvorrichtung eines Blutentnahmesystems, das folgende Schritte umfasst:
- Koppeln einer externen Komponente mit der Lanzette über ein bewegliches Kopplungselement;
- Auslösen eines Lanzettenausstoßmechanismus durch Betätigen der externen Komponente; und
- Ausstoßen der Lanzette.

12. Verfahren nach Anspruch 11, wobei das Kopplungselement eine Eingangskraft in eine Ausgangskraft umwandelt, die auf die Lanzette oder auf ein Vorspannelement einwirkt.

13. Verfahren nach Anspruch 11 oder 12, wobei ein erster Arm des Kopplungselements in eine erste Richtung bewegt wird und ein zweiter Arm des Kopplungselements in eine zweite Richtung bewegt wird.

14. Verfahren nach einem der Ansprüche 11 bis 13, wobei das Kopplungselement eine von einem Vorspannelement auf die Lanzette in ihrer Ausgangskonfiguration ausgeübte Anfangskraft bei Auslösung des Lanzettenausstoßmechanismus verringert oder aufhebt.

## Revendications

1. Système de prélèvement sanguin (10) pour prélever du sang à des fins de diagnostic, comprenant :
un dispositif de base configuré pour maintenir une lancette structurellement distincte (20) ;
le dispositif de base comprenant un mécanisme d'éjection de lancette actionnable pour éjecter la lancette à partir du dispositif de base, dans lequel le mécanisme d'éjection de lancette comprend un élément de couplage mobile (30) ;
dans lequel l'élément de couplage (30) comprend une structure de couplage pour coupler la lancette (20) à un composant externe (40) par le biais de l'élément de couplage (30) et activer ensuite le mécanisme d'éjection de lancette en faisant fonctionner le composant externe (40).

2. Système de prélèvement sanguin (10) selon la revendication 1, dans lequel le système de prélèvement sanguin comprend en outre la lancette (20).

3. Système de prélèvement sanguin (10) selon l'une quelconque des revendications précédentes, dans lequel le dispositif de base comprend un réceptacle de lancette (36) pour tenir la lancette (20).

4. Système de prélèvement sanguin (10) selon l'une quelconque des revendications précédentes, dans lequel le dispositif de base comprend un logement (50), le logement (50) entourant le mécanisme d'éjection, le logement (50) comprenant en outre une ouverture de réception (52), l'ouverture de réception (52) étant configurée pour recevoir une partie du composant externe.

5. Système de prélèvement sanguin (10) selon l'une quelconque des revendications précédentes, dans lequel l'élément de couplage (30) comprend un pivot (31) pour rediriger un mouvement d'entrée linéaire ou rotatif du composant externe (40) et/ou pour transformer un mouvement d'entrée linéaire du composant externe (40) en un mouvement rotatif ou cisaillé de l'élément de couplage (30).

6. Système de prélèvement sanguin (10) selon la revendication 5, dans lequel l'élément de couplage (30) comprend deux bras (32, 33), dans lequel les bras sont connectés l'un à l'autre par le pivot (31) dans un angle a.

7. Système de prélèvement sanguin (10) selon la revendication 6, dans lequel un bras (33, 34) se couple de manière opérationnelle à la lancette (20) dans sa configuration initiale avant l'éjection.

8. Système de prélèvement sanguin (10) selon l'une quelconque des revendications précédentes, comprenant en outre un élément de tension préalable (60), dans lequel l'élément de tension préalable (60) exerce une force initiale sur la lancette (20) dans sa configuration initiale, dans lequel la force initiale est réduite ou retirée à l'activation du mécanisme d'éjection de lancette.

9. Système de prélèvement sanguin (10) selon l'une quelconque des revendications précédentes, dans lequel l'élément de couplage (30) comprend une structure de couplage pour coupler la lancette (20) à un composant externe (40), le composant externe (40) étant une bande-test ou une broche d'éjection.

10. Kit de pièces comprenant un système de prélèvement sanguin selon la revendication 9 et une bande-test ou une broche d'éjection.

11. Procédé pour éjecter une lancette d'un dispositif de base d'un système de prélèvement sanguin comprenant les étapes de :
- coupler un composant externe à la lancette par le biais d'un élément de couplage mobile ;
- activer un mécanisme d'éjection de lancette en faisant fonctionner le composant externe ; et
- éjecter la lancette.

12. Procédé selon la revendication 11, dans lequel l'élément de couplage transforme une force d'entrée en une force de sortie qui agit sur la lancette ou sur un élément de tension préalable.

13. Procédé selon l'une quelconque des revendications 11 ou 12, dans lequel un premier bras de l'élément de couplage est déplacé dans une première direction et un second bras de l'élément de couplage est déplacé dans une seconde direction.

14. Procédé selon l'une quelconque des revendications 11 à 13, dans lequel l'élément de couplage réduit ou retire une force initiale exercée par un élément de tension préalable sur la lancette dans sa configuration initiale à l'activation du mécanisme d'éjection de lancette.
